# EUROPEAN PATENT APPLICATION

(11) **EP 3 960 150 A1**
(43) Date of publication of application: **02.03.2022**
(21) Application number: 20193431.2
(22) Date of filing: 28.08.2020
(51) Int. Cl.: A61K 8/34, A61K 8/35, A61K 8/365, A61K 8/40, A61K 8/41, A61K 8/49, A61K 8/55, A61K 8/60, A61K 8/64, A61Q 7/00, A61Q 7/02

(54) **ACTIVE AGENT MODULATING THE ACTIVITY OF AN ION CHANNEL FOR USE IN HAIR GROWTH REGULATION**

(71) Applicant: Monasterium Laboratory Skin & Hair Research Solutions GmbH, 48149 Münster (DE)
(72) Inventor: PAULS, Ralf, 22339 Hamburg (DE); MARDARYEV, Andrej, St. Saviour JE2 7PD Jersey (GB)
(74) Representative: WSL Patentanwälte Partnerschaft mbB

(57) **Abstract**

In order to provide a new agent for actively controlling hair growth in subjects, the present invention suggests an active agent for use in hair growth regulation, particularly for use in the treatment of hair growth (stimulation or inhibition), wherein said active agent activates, enhances, inactivates, blocks or dampens the cellular response of the transient receptor potential ion channel TRPM5 or interferes with the expression of said ion channel. Furthermore, the present invention is directed to compositions for use as a cosmetic or medicament in the treatment of hair growth said composition comprising at least one of the aforementioned active agents and at least one auxiliary agent. In addition, a non-therapeutic method of hair growth regulation is disclosed, wherein an effective amount of at least one of the aforementioned active agents is administered to a subject.

## Description

The present invention is directed to an active agent for use in hair growth regulation, particularly for use in the treatment of hair growth, wherein said active agent modulates the activity of an ion channel, including but not limited to activation, enhancement, inactivation or blocking an ion channel or dampening the cellular response induced by an ion channel or interfering with the expression of said ion channel.

Further, the present invention is directed to a composition for use as a cosmetic or medicament in the treatment of hair growth said composition comprising at least one active agent that modulates the activity of an ion channel, including but not limited to activation, enhancement, inactivation or blocking an ion channel or dampening the cellular response induced by an ion channel or interfering with the expression of said ion channel.

In addition, the present invention is directed to a non-therapeutic method of hair growth regulation, wherein an effective amount of at least one active agent that modulates the activity of an ion channel, including but not limited to activation, enhancement, inactivation or blocking an ion channel or dampening the cellular response induced by an ion channel or interfering with the expression of said ion channel, is administered to a subject.

The formation of hair follicles occurs once in the lifetime of healthy mammals, with the number of hair follicles determined in utero. Hair follicle morphogenesis is the generation of the whole hair follicle structure from the epidermis and the mesoderm. During postnatal life the hair follicle undergoes life-long cyclical transformations where it progresses through stages of rapid growth (anagen), regression (catagen) and relative quiescence (telogen) classified by morphological indicators and molecular markers.

There are a number of common and rare diseases associated with the hair follicle cycle, such as non-scarring alopecia, scarring alopecia, chronic and acute hair shedding, hypertrichosis or hirsutism. Further, there are a number of other conditions, where the control of unwanted hair on the human body or the promotion of hair growth on areas of the human body is desired, such as with drug-induced hair loss (e.g. chemotherapy), radiation-induced hair loss (e.g. radiotherapy) and with unwanted hair growth due to drugs such as cyclosporine A or diazoxide.

There is a continuous need for active agents for actively controlling hair growth in subjects without undesired side effects.

The inventors of the present invention found that the transient receptor potential ion channel TRPM5 is expressed in hair follicles. Specifically, strong cytoplasmic expression was detected in the outer root sheath (ORS), whereas a weaker expression was found in the inner root sheath (IRS). The dermal sheath (DS) and the dermal papilla (DP) did not show TRPM5 expression.

"Ion channels" are pore-forming proteins located in biological membranes. Through the pores of said proteins ions may pass the membrane down their electrochemical gradient. By either opening or closing the pore ion channels are capable of gating and controlling the flow of ions across the membranes thereby modulating the intracellular concentrations of ions. Alteration of intracellular ion concentrations affect a plethora of cellular responses and processes including, but not limited to, e.g. growth, differentiation, survival, death, mediator release, immune mechanisms, etc.

"Transient receptor potential channels" (TRP channels) are a group of ion channels located mostly on the plasma membrane, and there are about 30 TRP channels including TRPC, TRPV, TRPM, TRPN, TRPA, TRPP and TRPML. Said ion channels have a relatively non-selective permeability to cations, including sodium, calcium and magnesium.

"TRPM5" is the official gene symbol for "transient receptor potential cation channel subfamily M member 5" and identifies the protein that is encoded by the TRPM5 gene in humans (NCBI Gene ID: 29850; HGNC: 14323; NCBI mRNA sequence: NM_014555.3; NCBI protein sequence: NP_055370.1; Status on June 7, 2020).

The inventors of the present application have found that the pheromones 2-heptanone (in the following referred to as "2-Hep") and 2,5-dimethylpyrazine (in the following referred to as "DMP"), both of which being agonists of the transient receptor potential ion channel TRPM5, causing the channel to be open, maintained *ex vivo* human hair growth. Indeed, the number of hair follicles which remained in anagen during the 7-day-long culturing was higher in the pheromone treated groups than in the control ones. In addition, treatment of hair follicles with 2-Hep resulted in the elevation of intrafollicular expression of AXIN2 (known promoter of hair growth) and the suppression of level of TGFβ2 (known inhibitor of hair growth).

*Vice versa,* inhibition of TRPM5 using the selective inhibitor triphenylphosphine oxide (TPPO) consistently promoted catagen in human HF organ culture. Likewise, specific knock-down of TRPM5 by RNA silencing (thereby mimicking of inhibition of TRPM5 coupled signaling) reduced cell proliferation in hair matrix and proximal bulb, characteristics of hair growth inhibition. In addition, knock-down of TRPM5 by RNA silencing induced a tendency of apoptosis. Finally, knock-down of TRPM5 by RNA silencing resulted in the suppression of intrafollicular expressions of LEF1 and IGF1 (known promoters of hair growth) and the elevation of levels of TGFβ2 and SFRP1 (known inhibitors of hair growth).

In addition to the above active agents, the inventors identified a number of additional active agents acting on TRPM5 and, thus, being suitable for use in the treatment of hair growth.

Concluding, the present invention is characterised by an active agent for use in the treatment of hair growth, wherein said active agent activates, enhances, inactivates or blocks TRPM5 or dampens the cellular response induced by the transient receptor potential ion channel TRPM5 or interferes with the expression of said ion channel.

According to one alternative of the present invention the active agent that activates, enhances, inactivates or blocks the ion channel or dampens the cellular response induced by the transient receptor potential ion channel TRPM5 is an agonist or antagonist of said ion channel.

As referred to herein an "agonist" of the transient receptor potential ion channel TRPM5 is a substance that binds to said ion channel and activates or enhances the ion channel thereby augmenting the cellular response linked to TRPM5.

As referred to herein an "antagonist" of the transient receptor potential ion channel TRPM5 is a substance that binds to said ion channel and blocks TRPM5 thereby dampening the cellular response to an agonist rather than activating or enhancing it like an agonist.

An "inverse agonist" of the transient receptor potential ion channel TRPM5 is a substance that binds to said ion channel and in turn induces an opposite the cellular response than seen when applying an agonist.

With respect to the transient receptor potential ion channel TRPM5 of the present invention the term "cellular response" is primarily to be understood as a change of ion concentration in the cell being the result of an agonist/antagonist/inverse agonist binding to the transient receptor potential ion channel TRPM5. However, the term also encompasses instances, where the binding of an agonist/antagonist/inverse agonist induces a cellular response without altering the intracellular ion concentration.

Accordingly an inventive agonist of the transient receptor potential ion channel TRPM5 activates or enhances said ion channel to produce the cellular response like an endogenous agonist does, whereas the inventive antagonist of said ion channel blocks or dampens the ion channel to produce the cellular response as usually activated/enhanced by an endogenous agonist, and the inventive inverse agonist modulates the ion channel to produce the opposite cellular response as usually activated/enhanced by endogenous agonists.

In those embodiments, where the active agent is an agonist of the transient receptor potential ion channel TRPM5, said active agent is used in the treatment of unwanted hair loss. In those embodiments, where the active agent is an antagonists/inverse agonists of the transient receptor potential ion channel TRPM5, said active agent is used in the treatment of unwanted hair growth.

In specific embodiments of the invention the ion channel agonist/antagonist/inverse agonist used is a TRPM5 agonist selected from the group consisting of dimethylpyrazine, dimethylethylpyrazine, tetramethylpyrazine, 2-heptanone, eugenol, SID2848719 (CAS number 702636-90-6, SMILES, NC(=O)C1(CCN(CC1)S(=O)(=O)c1ccc20CCCOc2c1)N1CCCCC1), rutamarin, bergapten, xanthotoxin, isopimpinellin, carbachol, 3-deoxyglucosone, glucagon-like peptide 1, (E)-N-(3,4dimethoxybenzylidene)-2-naphthalene-1-yl)acetohydrazide, or a combination thereof. Alternatively, the TRPM5 agonist is an aptamer binding to the TRPM5 ion channel and activating or enhancing the ion channel to produce the cellular response.

The term "aptamer" as used herein refers to DNA, RNA or XNA oligonucleotide or peptide molecules that bind to a specific target molecule, such as ion channel molecules.

In other embodiments of the invention the ion channel agonist/antagonist/inverse agonist used is a TRPM5 antagonist/inverse agonist selected from triphenylphosphine oxide, econazole, miconazole, chlorpromazine, or combinations thereof. Alternatively, the TRPM5 antagonist/inverse agonist is an aptamer binding to the TRPM5 ion channel and inactivating, blocking or dampening the ion channel to produce the cellular response.

According to an alternative of the present invention, an active agent that interferes with the expression of the ion channel TRPM5 is used in hair growth regulation. An active agent that interferes with the expression of the ion channel TRPM5 is either miRNA, siRNA or a ribozyme targeted to the TRPM5 gene or targeted to the mRNA corresponding to the TRPM5 gene.

The term "miRNA" refers to microRNA, i.e. small non-coding RNA molecules containing 21 to 23 nucleotides and functioning in RNA knock-down and post-transcriptional regulation of gene expression. miRNAs function via base-pairing with complementary sequences within mRNA molecules. As a result, these mRNA molecules are knocked-down, by cleavage, destabilization and/or less efficient translation of the mRNA.

The term "siRNA" refers to small interfering RNA, i.e. small non-coding RNA molecules, 20 to 25 base pairs in length. siRNA interferes with the expression of specific genes with complementary nucleotide sequences by degrading mRNA after transcription, thereby preventing translation.

According to the present invention a gene is "targeted" by a miRNA, siRNA or a ribozyme when the miRNA, siRNA or a ribozyme molecule selectively decreases or inhibits the expression of TRPM5. The phrase "selectively decrease or inhibit" as used herein refers to miRNA, siRNA or a ribozyme that affects the expression of TRPM5.

In specific embodiments of the invention miRNA or siRNA interfere with the gene expression of the ion channel TRPM5 by hybridizing under stringent conditions to the gene transcript, i.e. the TRPM5 mRNA, wherein hybridizing "under stringent conditions" means annealing to the target mRNA region, under standard conditions, e.g., high temperature (e.g. < 60 °C for 2 hours) and/or low salt content (e.g. 0.1xSSC) which tend to disfavor hybridization.

According to the present invention one of the above-mentioned active agents is used in the treatment of hair growth. In specific embodiments of the invention the treatment is effected locally, i.e. in, on or at the skin area to be treated. In some embodiments of the invention said treatment is effected topically, wherein the term "topical" refers to a medication that is applied to a particular place on the skin and/or its appendages, such as hair. In specific embodiments the topical application is epicutaneous, meaning that the agonist or antagonist is applied directly to the skin. In other embodiments of the invention the treatment is effected transdermally, wherein the term "transdermal" refers to a medication that is applied across the Stratum corneum into the deeper skin layers, e.g. by injection with a standard needle or microneedles. In other embodiments of the invention the treatment is effected transappendageally, wherein the term "transappendageal" refers to an applied medication that is permeating the skin via skin appendage structures (such as the hair follicles, sebaceous glands, and sweat glands) into the deeper skin layers.

The term "treatment" as used herein refers to any action resulting in the change of a physical condition. Particularly, the "treatment of hair growth" refers to any change of an initial hair growth condition, such as unwanted presence of hair, unwanted lack of hair, unwanted slow/fast hair growth, chemotherapy-related hair loss, drug-related hair growth and radiation-related hair loss. In other words, the present invention is directed to any kind of hair growth regulation.

In specific embodiments of the invention the above-mentioned active agent is used as a cosmetic in the treatment of hair growth. Particularly, the use as a cosmetic occurs non-therapeutically, but in order to achieve a change of an initial hair growth condition, such as unwanted presence of hair, unwanted lack of hair, unwanted slow/fast hair growth, wherein said initial condition is not caused by a disease or disorder.

In those embodiments, where the above mentioned active agent is used as a cosmetic the active agent used should be cosmetically acceptable, wherein "cosmetically acceptable" means that the active agent should not be toxic or harmful or have any other detrimental side effects upon application on hair and/or skin.

In other specific embodiments of the invention the above mentioned active agent is used as a medicament in the topical treatment of hair growth disorder, wherein the term "disorder" refers to any functional abnormality or disturbance of the normal healthy condition, and the term "medicament" refers to a substance useful in curing, treating or preventing a condition of disorder.

In some embodiments the above mentioned active agent is used as a medicament in the topical treatment of a hair growth disorder being selected from the group consisting of nonscarring (non cicatricial) alopecia, such as alopecia areata, telogen effluvium, androgenetic alopecia, anagen effluvium, loose anagen syndrome and female pattern hair loss; scarring (cicatricial) alopecia, such as cutaneous lichen planopilaris, frontal fibrosing alopecia, discoid lupus erythematosus, dissecting cellulitis and folliculitis decalvans; and excessive hair growth such as hypertrichiosis and hirsutism.

In those embodiments, where the above mentioned active agent is used as a medicament the active agent used should be pharmaceutically acceptable, wherein "pharmaceutically acceptable" means that the active agent should not be toxic or harmful or have any other detrimental side effects upon application on hair and/or skin.

In some embodiments at least one of the inventive active is used as an ingredient of a composition for use as a cosmetic or medicament in the topical treatment of hair growth said composition further comprising at least one auxiliary agent selected from the group consisting of carriers, excipients, adjuvants, diluents, and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

In the inventive compositions the concentration of the active agent usually is in the range of from 10 to 10.000 µM. In some embodiments the lower limit for the concentration of the active agent is 30 µM or even 100 µM. In some embodiments the upper limit is 3.000 µM or 1.000 µM. This results in preferred ranges of e.g. from 30 to 10.000 µM, from 30 to 3.000 µM, from 10 to 3.000 µM, 100 to 3.000 µM and the like.

In specific embodiments such compositions further comprise at least one other active agent being effective in the treatment of hair growth.

In such embodiments the other active agent may be selected from hair growth inhibitors, such as inhibitors of ornithine decarboxylase (including Difluoromethylornithine (DFMO)), antiandrogen compounds, inhibitors of 5-α-reductase, inhibitors of androgen ion channel, inhibitors of S-adenosyl methionine decarboxylase, inhibitors of γ-glutamyl transpeptidase, inhibitors of adenylosuccinate synthetase, inhibitors of aspartate transcarbamylase, inhibitors of transglutaminase, inhibitors of L-asparagine synthetase, pantothenic acid and its analogues, sulfhydryl reactive compounds, inhibitors of lipoxygenase, inhibitors of cyclooxygenase, inhibitors of nitric oxide synthetase, inhibitors of ornithine amino transferase, inhibitors of cysteine synthesis pathway enzymes, inhibitors of protein kinase C, catechin compounds, green tea polyphenols, non-steroidal angiogenesis suppressors, inhibitors of arginase, inhibitors of the metabolic pathway for the conversion of glucose to acetyl-CoA, compounds that inhibit the formation of glycoprotein, proteoglycans, and glycosaminogly-cans, inhibitors of matrix metalloproteinase, inhibitors of the cholesterol synthesis pathway, inhibitors of DNA topoisomerase, inhibitors of aminoacyl-tRNA synthetase, inhibitors of the hypusine biosynthesis pathway, compounds that activate androgen conjugation, inhibitors of alkaline phosphatase, inhibitors of protein tyrosine kinase, and compounds that increase cellular ceramide levels. Specific examples include cyproterone acetate, progesterone, acivicin, anthglutin, L-alanosine, guanidino-succinic acid, ethacrynic acid, D-pantothenic acid, pantoyl alcohol, gabaculin, canaline, isonicotinic acid, verapamil, phentolamine, pentosan polysulfate, nafoxidine, tripelennamine, oc-tapine, phloretin, argaric acid, simvastatin, atorvastatin, lovastatin, fluvastatin, mevastatin, N^{G}-methyl-L-arginine, NG-nitro-L-arginine, benzoyl-L-argininamide, L-argininamide, quercetin, apigenin, nordihydroguaratic acid (NDGA), ketoprofen, naproxen, tolmetin, diclofenac, diflunisal, sulindac, thiosalicylic acid, cysteamine, diethyldithiocarbamic acid, D-penicillamine, N-acetyl-L-cysteine, bathocuproine, enalapril, tamoxifen, cimetidine, mycophenolic acid, tetracycline, doxycycline, minocycline, thioridazine, trifluoperizine, 1-(5-isoquinolinylsulfonyl)-2-methylpiperazine, glycyrrhetinic acid, epigallocatechin gallate, epicatechin gallate, epigallocatechin, epicatechin, fusidic acid, and nitroso-acetyl-penicillamine.

In yet other embodiments the other active agent may be selected from preventatives of chemotherapy- or radiation-induced alopecia or hair loss, such as 4-((cyanoimino((1,2,2-trimethylpropyl)amino)methyl)amino)benzonitrile, epidermal growth factor, fibroblast growth factors, including but not limited to keratinocyte growth factor (FGF7), prostaglandins, cyclin dependent kinases, p53 inhibitors, capase-3 inhibitors, N-acyl cysteine, parathyroid hormone antagonist, alpha-tocopherol, cyclosporine, angiotensin ion channel blockers, and minoxidil.

Generally, the inventive composition may be used in any formulation suitable for treatment of hair growth. In specific embodiments of the invention the composition is formulated in the form of an ointment, a lotion, a cream, a shampoo, a gel, a spray, a plaster or a sustained release plaster. In other specific embodiments of the invention the composition is formulated in the form of a solution. Said solution may be applied by means of a microneedle device or prior to sonication, electrical stimulation, etc.

As mentioned above, the inventive use of the above mentioned active agent may also occur non-therapeutically, wherein non-therapeutically refers to a treatment not being directed to curing, treating or preventing a condition of disorder (see above).

Therefore, the present invention is further directed to a non-therapeutic method of hair growth regulation, wherein an effective amount of at least one of the above mentioned active agent is administered to a subject.

The non-therapeutic method also encloses embodiments, where the above mentioned active agent is administered to the subject to be treated simultaneously, sequentially or separately together with at least one other active agent useful in the treatment of hair growth (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: is a photograph showing the result of an immunofluorescence analysis of TRPM5 expression in human skin,
- Figure 2: is a photograph showing the result of an immunofluorescence analysis of TRPM5 expression in human skin,
- Figure 3: is a graph showing that TRPM5 protein expression is significantly reduced in TRMP5-depleted human hair follicles,
- Figure 4: is a graph showing decreased cell proliferation (Ki67) in siTRPM5-treated hair follicles,
- Figure 5: is a graph showing increased apoptosis (TUNEL) in siTRPM5-treated hair follicles,
- Figure 6: is a graph showing that TRPM5 siRNA reduces expression levels of LEF1 and IGF1 in human hair follicles,
- Figure 7: is a graph showing that TRPM5 siRNA increases expression of TGFB2 and SFRP1 in human hair follicles,
- Figure 8: is a graph showing that 2,5-dimethylpyrazine (DMP) prolongs anagen in human hair follicle organ culture,
- Figure 9: is a graph showing that 2-heptanone (2-Hep) prolongs anagen in human hair follicle organ culture,
- Figure 10: is a graph showing that expression of catagen-promoting gene TGFB2 was down-regulated in 2-Hep-treated hair follicles, while Wnt target gene AXIN2 up-regulated,
- Figure 11: is a graph showing that triphenyl phosphine oxide (TPPO) promotes catagen in human hair follicle organ culture, and
- Figure 12: is a graph showing that Expression levels of of IGF1 and FGF7 genes were significantly down-regulated in TPPO-treated hair follicles, while TGFB2 transcript was up-regulated.

### Examples

### 1. Immunofluorescence analysis of TRPM5 expression in human skin

Cryosection without fixation was dried and then fixed in acetone at -20°C. Slides were then washed in Phosphate-buffered saline (PBS). Sample sections were pre-treated with goat serum. anti-TRPM5 was added to sample sections and incubated. Slides were washed in PBS and goat anti-rabbit-A488 antibody was added to sample sections and incubated. Slides were washed in PBS and sample sections were incubated with DAPI. Slides were washed again in PBS and mounted with coverslip using Southernbiotech Fluoromount.

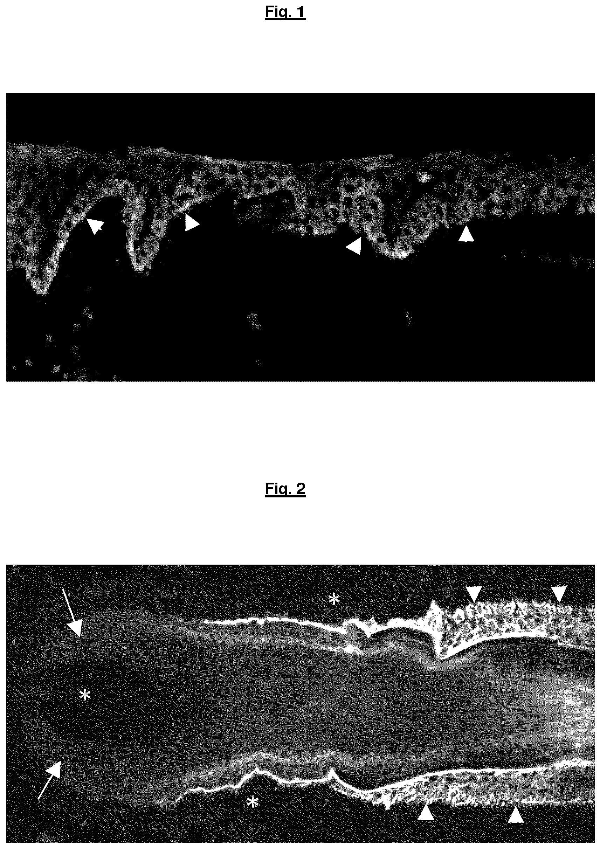

Figures 1 and 2 show the result of the immunofluorescence analysis of TRPM5 expression in human skin according to the above protocol. From Figure 1 it can be identified that TRPM5 protein expression is predominately detected in the basal layer of the epidermis (arrowheads). From Figure 2 it can be identified that strong TRPM5 expression is detected in the outer root sheath (arrowheads), with weaker expression in the hair matrix (arrows), while no expression was detected in the hair follicle mesenchyme (asterisks).

### 2. Inactivation of TRPM5 by RNAi knockdown

For analysing the effect of inactivating TRPM5 by RNAi knock-down, human hair follicles were transfected with 1µM TRPM5-targeting Accell siRNA (siTRPM5) and scrambled siRNA (siScr). After 24 hours, transfected hair follicles were snap frozen in liquid nitrogen for immuno-fluorescent analysis or collected in RNA extraction buffer for RNA isolation. TRPM5 pro-tein expression was detected as above. Ki67 protein expression was detected as for TRPM5 using mouse anti-human Ki67 primary antibody and goat anti-mouse-A568 sec-ondary antibody. For TUNEL immunolabelling, hair follicle cryosections were fixed in formalin/ethanol/acetic acid and labeled with digoxigenin-deoxyUTP (ApopTag Fluorescein In Situ Apoptosis detection kit; Millipore) in the presence of terminal deoxynucleo-tidyl transferase according to the manufacturers protocol. TUNEL+ cells were visualized with anti-digoxigenin FITC-conjugated antibody (ApopTag kit). Cells positive for Ki-67 or TUNEL were counted per hair matrix and ORS and were normalized to the total number of nuclei (DAPI+).

Total RNA was isolated using PicoPure RNA Isolation Kit (Applied BioSystems) according to the manufacturer's protocol. After isolation, 100 ng of total RNA was reverse-transcribed into cDNA using a Tetro cDNA synthesis kit (Bioline). Quantitative-PCR was performed using the StepOne Plus real time PCR system (Applied Biosystems) using Taqman fast Advanced master mix and Taqman probes for LEF1, IGF1, TGFB2 AND SFRP1 (Applied Biosystems). The amount of the aforementioned transcripts was normal-ized to the expression of reference gene (GAPDH) using the ΔΔCt method. Results were performed in triplicate for each experiment.

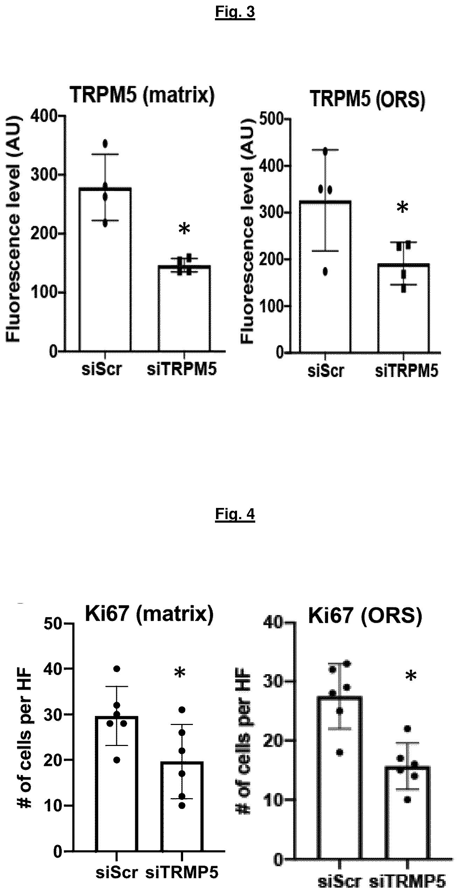

Figures 3 - 6 show the results of siRNA targeting TRPM5 as mentioned above (Mean+/- sd, Mann Whitney U test, *p<0.05, **p<0.001).

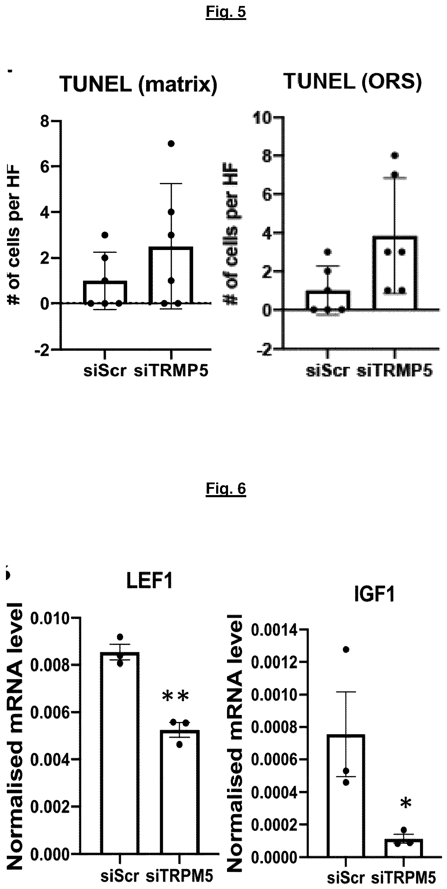

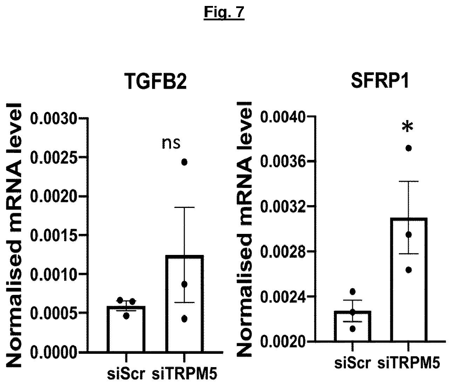

According to Figure 3 TRPM5 protein expression is significantly reduced in TRMP5-depleted human hair follicles. Figures 4 and 5 show decreased cell proliferation (Ki67) and increased apoptosis (TUNEL) in siTRPM5-treated hair follicles. According to Figures 6 and 7 TRPM5 siRNA reduces expression levels of LEF1 and IGF1 (Fig. 6), while increasing expression of TGFB2 and SFRP1 (Fig. 7) in human hair follicles.

### 3. Activation of TRPM5 with 2,5-dimethylpyrazine (DMP)

Human anagen hair follicles were isolated from the skin of males undergoing hair transplantation with written informed consent. Microdissected hair follicles were amputated at the level of dermal-subcutaneous junction and equilibrated in Williams' E medium (WEM) (Life Technologies) supplemented with 2 mM L-glutamine (Life Technologies), 10 ng/ml hydrocortisone, 10 µg/ml insulin, and antibiotics (all from Sigma-Aldrich). After 24 hours, growing undamaged anagen hair follicles were selected for treatment with DMP Human hair follicles were cultured in presence of DMP (12.5 µM). The culture medium was changed every two days with addition of DMP. Hair follicles were photographed and percentage of hair follicles at different stages of hair cycle were calculated at day 3 and day 6 of the treatment and compared to vehicle.

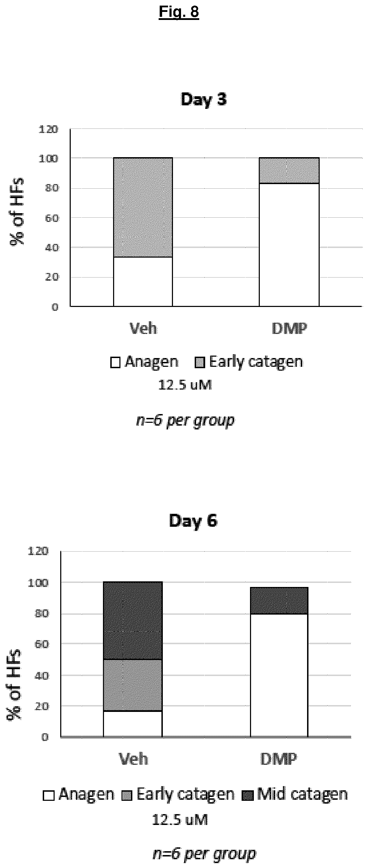

According to the graph shown in Figure 8 the TRPM5 agonist 2,5-dimethylpyrazine (DMP) prolongs anagen in human hair follicle organ culture as compared to vehicle (Veh).

### 4. Activation of TRPM5 with 2-heptanone (2-Hep)

Human anagen hair follicles were isolated from the skin of males undergoing hair transplantation with written informed consent. Microdissected hair follicles were amputated at the level of dermal-subcutaneous junction and equilibrated in Williams' E medium (WEM) (Life Technologies) supplemented with 2 mM L-glutamine (Life Technologies), 10 ng/ml hydrocortisone, 10 µg/ml insulin, and antibiotics (all from Sigma-Aldrich). After 24 hours, growing undamaged anagen hair follicles were selected for treatment with 2-Hep (12.5 µM).

The culture medium was changed every two days with addition of 2-Hep. Hair follicles were photographed and percentage of hair follicles at different stages of hair cycle were calculated at day 3 and day 6 of the treatment and compared to vehicle.

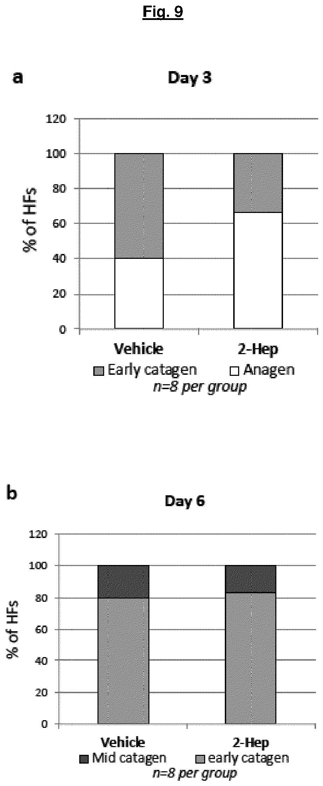

According to the graph shown in Figure 9 the TRPM5 agonist 2-heptanone (2-Hep) prolongs anagen in human hair follicle organ culture.

For gene expression analysis, human hair follicles were microdissected as above and treated with 2-Hep (12.5uM) for 6 hours. Following the treatment, total RNA was isolated and processed for cDNA synthesis as above. Quantitate PCR was performed using Taman assay with probes for TGFB2 and AXIN2 (Applied Biosystems).

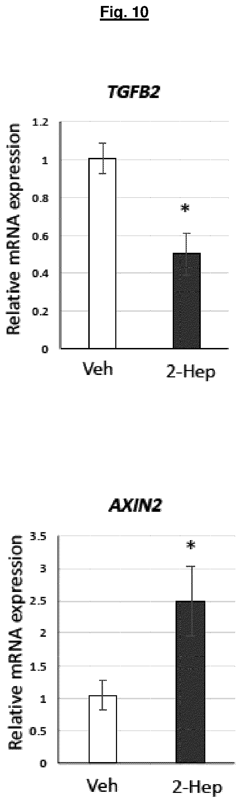

Figure 10 is a graph showing that expression of catagen-promoting gene TGFB2 was down-regulated in 2-Hep-treated hair follicles as compared to vehicle (Veh), while Wnt target gene AXIN2 up-regulated (Mean+/- sd, Mann Whitney U test, *p<0.05).

### 5. Inactivation of TRPM5 with Triphenyl phosphine oxide (TPPO)

Human anagen hair follicles were isolated from the skin of males undergoing hair transplantation with written informed consent. Microdissected hair follicles were amputated at the level of dermal-subcutaneous junction and equilibrated in Williams' E medium (WEM) (Life Technologies) supplemented with 2 mM L-glutamine (Life Technologies), 10 ng/ml hydrocortisone, 10 µg/ml insulin, and antibiotics (all from Sigma-Aldrich). After 24 hours, growing undamaged anagen hair follicles were selected for treatment withHuman hair follicles were cultured in presence of TPPO (150 µM). The culture medium was changed every two days with addition of TPPO. Hair follicles were photographed and percentage of hair follicles at different stages of hair cycle were calculated at day 3 and day 6 of the treatment and compared to vehicle.

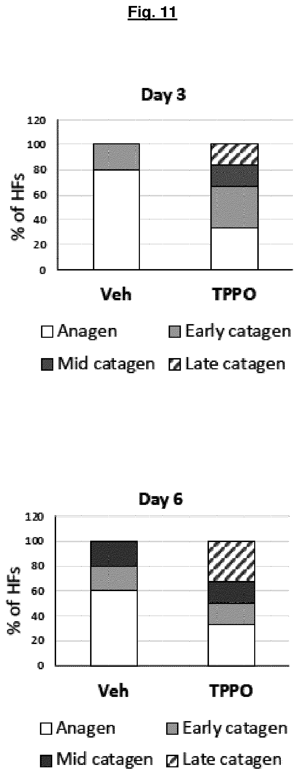

According to the graph shown in Figure 11 the TRPM5 antagonist triphenyl phosphine oxide (TPPO) promotes catagen in human hair follicle organ culture as compared to vehicle (Veh)

For gene expression analysis, human hair follicles were microdissected as above and treated with TPPO (150uM) for 6 hours. Following the treatment, total RNA was isolated and processed for cDNA synthesis as above. Quantitate PCR was performed using Taman assay with probes for IGF1, FGF7 and TGFB2 (Applied Biosystems).

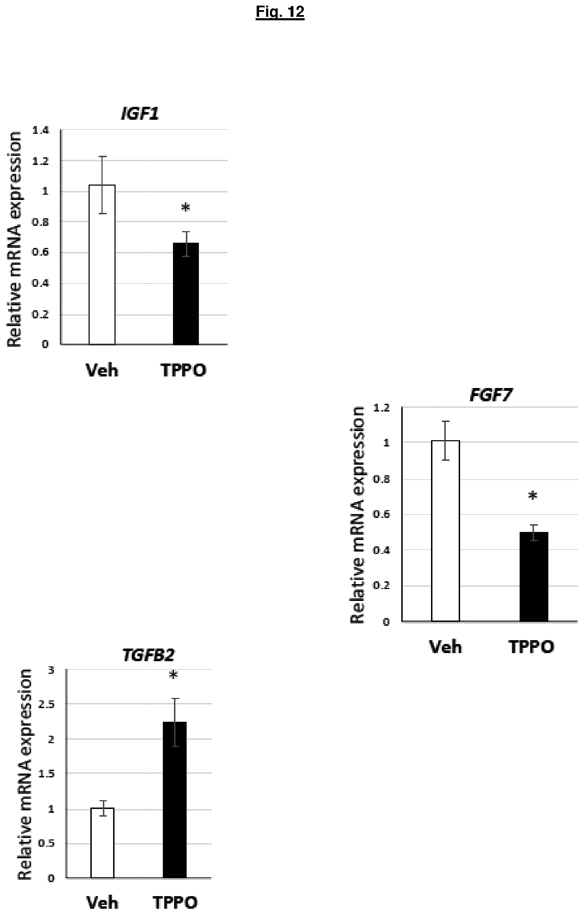

Figure 12 is a graph showing that expression levels of of IGF1 and FGF7 genes were significantly down-regulated in TPPO-treated hair follicles as compared to vehicle (Veh), while TGFB2 transcript was up-regulated (Mean+/- SEM, Mann Whitney U test, *p<0.05).

## Claims

1. Active agent for use in the treatment of hair growth, wherein said active agent activates, enhances, inactivates, blocks or dampens the cellular response of the transient receptor potential ion channel TRPM5 or interferes with the expression of said ion channel.

2. Active agent for use in the treatment of hair growth according to claim 1, wherein the active agent is an agonist of the transient receptor potential ion channel TRPM5 for use in the treatment of unwanted hair loss.

3. Active agent for use in the treatment of hair growth according to claim 1, wherein the active agent is an antagonists/inverse agonists of the transient receptor potential ion channel TRPM5 for use in the treatment of unwanted hair growth.

4. Active agent for use in the treatment of hair growth according to one of the claims 1 to 3, wherein the active agent is
a) any one of the TRPM5 activating agonists dimethylpyrazine, dimethylethylpyrazine, tetramethylpyrazine, 2-heptanone, eugenol, SID2848719 (CAS number 702636-90-6), rutamarin, bergapten, xanthotoxin, isopimpinellin, carbachol, 3-deoxyglucosone, glucagon-like peptide 1, (E)-N-(3,4dimethoxybenzylidene)-2-naphthalene-1-yl) acetohydrazide or a combination thereof, or an aptamer binding to TRPM5 and activating or enhancing said ion channel to produce a cellular response, or
b) any one of the TRPM5 inactivating antagonists/inverse agonists triphenylphosphine oxide, econazole, miconazole, chlorpromazine or a combination thereof, or an aptamer binding to TRPM5 and inactivating, blocking or dampening said ion channel to produce a cellular response.

5. Active agent for use in the treatment of hair growth according to claim 1, wherein the active agent is miRNA, siRNA or a ribozyme targeted to TRPM5.

6. Active agent according to one of the claims 1 to 5 for use as a cosmetic in the treatment of hair growth.

7. Active agent according to one of the claims 1 to 5 for use as a medicament in the treatment of hair growth disorder.

8. Active agent for use as a medicament according to claim 7, wherein the hair growth disorder to be treated is nonscarring (non cicatricial) alopecia, such as alopecia areata, telogen effluvium, androgenetic alopecia,anagen effluvium, loose anagen syndrome and female pattern hair loss; scarring (cicatricial) alopecia, such as cutaneous lichen planopilaris, frontal fibrosing alopecia, discoid lupus erythematosus, dissecting cellulitis and folliculitis decalvans; and excessive hair growth such as hypertrichiosis and hirsutism.

9. Composition for use as a cosmetic or medicament in the treatment of hair growth said composition comprising at least one active agent according to one of the claims 1 to 8 and at least one auxiliary agent selected from the group consisting of carriers, excipients, adjuvants, diluents, and disintegrants.

10. Composition for use as a cosmetic or medicament in the treatment of hair growth according to claim 9, wherein the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

11. Composition for use as a cosmetic or medicament in the treatment of hair growth according to claim 9 or claim 10, further comprising at least one other active agent being effective in the treatment of hair growth.

12. Composition for use as a cosmetic or medicament in the treatment of hair growth according to one of the claims 9 to 11, wherein the composition is formulated in the form of an ointment, a lotion, a cream, a shampoo, a hair conditioner, a gel, a solution, a spray, a plaster or a sustained release plaster.

13. Non-therapeutic method of hair growth regulation, wherein an effective amount of at least one active agent that activates, enhances, inactivates, blocks or dampens the cellular response of the transient receptor potential ion channel TRPM5 or interferes with the expression of said ion channel is administered to a subject.

14. Non-therapeutic method of hair growth regulation according to claim 13, wherein the active agent is
a) any one of the TRPM5 activating agonists dimethylpyrazine, dimethylethylpyrazine, tetramethylpyrazine, 2-heptanone, eugenol, SID2848719 (CAS number 702636-90-6), rutamarin, bergapten, xanthotoxin, isopimpinellin, carbachol, 3-deoxyglucosone, glucagon-like peptide 1, (E)-N-(3,4dimethoxybenzylidene)-2-naphthalene-1-yl) acetohydrazide or a combination thereof, or an aptamer binding to TRPM5 and activating or enhancing said ion channel to produce a cellular response, or
b) any one of the TRPM5 inactivating antagonists/inverse agonists triphenylphosphine oxide, econazole, miconazole, chlorpromazine or a combination thereof, or an aptamer binding to TRPM5 and inactivating, blocking or dampening said ion channel to produce a cellular response.

15. Non-therapeutic method of hair growth regulation according to one of the claims 13 and 14, wherein the active agent is miRNA, siRNA or a ribozyme targeted to the TRPM5 gene or targeted to the mRNA corresponding to the TRPM5 gene.

## Amended claims

### Amended claims in accordance with Rule 137(2) EPC.

1. Active agent for therapeutical use in the treatment of hair growth or active agent for non-therapeutical use in the treatment of hair growth, wherein said active agent activates, enhances, inactivates, blocks or dampens the cellular response of the transient receptor potential ion channel TRPM5 or interferes with the expression of said ion channel, and wherein
a) in the treatment of unwanted hair loss the active agent is selected from any one of the TRPM5 activating agonists dimethylpyrazine, dimethylethylpyrazine, 2-heptanone, SID2848719 (CAS number 702636-90-6), rutamarin, xanthotoxin, isopimpinellin, carbachol, 3-deoxyglucosone, (E)-N-(3,4dimethoxybenzylidene)-2-naphthalene-1-yl) acetohydrazide or a combination thereof, or an aptamer binding to TRPM5 and activating or enhancing said ion channel to produce a cellular response, or
b) in the treatment of unwanted hair growth the active agent is selected from any one of the TRPM5 inactivating antagonists/inverse agonists triphenylphosphine oxide, econazole, miconazole, chlorpromazine or a combination thereof, or an aptamer binding to TRPM5 and inactivating, blocking or dampening said ion channel to produce a cellular response or
c) the active agent is selected from miRNA, siRNA or a ribozyme targeted to TRPM5.

2. Active agent for therapeutical use in the treatment of hair growth or active agent for non-therapeutical use in the treatment of hair growth according to claim 1, wherein the active agent is an agonist of the transient receptor potential ion channel TRPM5 for use in the treatment of unwanted hair loss.

3. Active agent for therapeutical use in the treatment of hair growth or active agent for non-therapeutical use in the treatment of hair growth according to claim 1, wherein the active agent is an antagonists/inverse agonists of the transient receptor potential ion channel TRPM5 for use in the treatment of unwanted hair growth.

4. Use of an active agent according to one of the claims 1 to 5 as a cosmetic for the non-therapeutical treatment of hair growth.

5. Active agent according to one of the claims 1 to 5 for therapeutical use in the treatment of hair growth disorder.

6. Active agent for therapeutical use according to claim 7, wherein the hair growth disorder to be treated is nonscarring (non cicatricial) alopecia, such as alopecia areata, telogen effluvium, androgenetic alopecia,anagen effluvium, loose anagen syndrome and female pattern hair loss; scarring (cicatricial) alopecia, such as cutaneous lichen planopilaris, frontal fibrosing alopecia, discoid lupus erythematosus, dissecting cellulitis and folliculitis decalvans; and excessive hair growth such as hypertrichiosis and hirsutism.

7. Medical composition for therapeutical use in the treatment of hair growth said composition comprising at least one active agent according to one of the claims 1 to 8 and at least one auxiliary agent selected from the group consisting of carriers, excipients, adjuvants, diluents, and disintegrants.

8. Medical composition for therapeutical use in the treatment of hair growth according to claim 9, wherein the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

9. Medical composition for therapeutical use in the treatment of hair growth according to claim 9 or claim 10, further comprising at least one other active agent being effective in the treatment of hair growth.

10. Medical composition for therapeutical use in the treatment of hair growth according to one of the claims 9 to 11, wherein the composition is formulated in the form of an ointment, a lotion, a cream, a shampoo, a hair conditioner, a gel, a solution, a spray, a plaster or a sustained release plaster.

11. Cosmetic hair growth composition for non-therapeutical use in the treatment of hair growth said composition comprising at least one active agent according to one of the claims 1 to 8 and at least one auxiliary agent selected from the group consisting of carriers, excipients, adjuvants, diluents, and disintegrants.

12. Cosmetic hair growth composition for non-therapeutical use in the treatment of hair growth according to claim 9, wherein the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

13. Cosmetic hair growth composition for non-therapeutical use in the treatment of hair growth according to claim 9 or claim 10, further comprising at least one other active agent being effective in the treatment of hair growth.

14. Cosmetic hair growth composition for non-therapeutical use in the treatment of hair growth according to one of the claims 9 to 11, wherein the composition is formulated in the form of an ointment, a lotion, a cream, a shampoo, a hair conditioner, a gel, a solution, a spray, a plaster or a sustained release plaster.

15. Non-therapeutic method of hair growth regulation, wherein an effective amount of at least one active agent that activates, enhances, inactivates, blocks or dampens the cellular response of the transient receptor potential ion channel TRPM5 or interferes with the expression of said ion channel is administered to a subject, wherein
a) in the treatment of unwanted hair loss the active agent is selected from any one of the TRPM5 activating agonists dimethylpyrazine, dimethylethylpyrazine, 2-heptanone, SID2848719 (CAS number 702636-90-6), rutamarin, xanthotoxin, isopimpinellin, carbachol, 3-deoxyglucosone, (E)-N-(3,4dimethoxybenzylidene)-2-naphthalene-1-yl) acetohydrazide or a combination thereof, or an aptamer binding to TRPM5 and activating or enhancing said ion channel to produce a cellular response, or
b) in the treatment of unwanted hair growth the active agent is selected from any one of the TRPM5 inactivating antagonists/inverse agonists triphenylphosphine oxide, econazole, miconazole, chlorpromazine or a combination thereof, or an aptamer binding to TRPM5 and inactivating, blocking or dampening said ion channel to produce a cellular response or
c) the active agent is selected from miRNA, siRNA or a ribozyme targeted to the TRPM5 gene or targeted to the mRNA corresponding to the TRPM5 gene.
